Europäisches Patentamt

European Patent Office

Office européen des brevets

(19)

(11) Veröffentlichungsnummer: **0 210 424 B1**

(12) **EUROPÄISCHE PATENTSCHRIFT**

(45) Veröffentlichungstag der Patentschrift: **19.08.92**

(51) Int. Cl.5: **A61M 5/148**

(21) Anmeldenummer: **86108471.3**

(22) Anmeldetag: **20.06.86**

(54) **Behälter aus flexiblem Material zur Aufnahme eines aufblasbaren Druckkissens und eines Infusions/Transfusions-Beutels.**

(30) Priorität: **31.07.85 DE 3527498**

(43) Veröffentlichungstag der Anmeldung:
**04.02.87 Patentblatt 87/06**

(45) Bekanntmachung des Hinweises auf die
Patenterteilung:
**19.08.92 Patentblatt 92/34**

(84) Benannte Vertragsstaaten:
**AT BE CH FR GB IT LI LU NL SE**

(56) Entgegenhaltungen:
**EP-A- 0 102 012**
**US-A- 4 090 514**
**US-A- 4 379 453**

(73) Patentinhaber: **TRANSMED Medizintechnik GmbH**
**Zinsdorfer Weg 16**
**W-4798 Wünnenberg(DE)**

(72) Erfinder: **Zoratto, Elke**
**Kontumazgarten 24**
**W-8500 Nürnberg 80(DE)**

(74) Vertreter: **Kehl, Günther, Dipl.-Phys. et al**
**Patentanwälte Hagemann & Kehl Ismaninger**
**Strasse 108 Postfach 86 03 29**
**W-8000 München 86(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft einen Behälter aus flexiblem Material zur Aufnahme eines aufblasbaren Druckkissens und eines Infusions/Transfusions-Beutels zur Durchführung einer Infusion/Transfusion unter Druck nach dem Oberbegriff des Anspruchs 1

Ein Behälter dieser Art ist aus den Unterlagen des deutschen Gebrauchsmusters 70 47 574 bekannt. Bei diesem bekannten Behälter wird der Transfusionsbeutel in eine sackartige, d.h. an drei Seiten verschlossene und nur an einer Seite offene Tasche gesteckt, die an einer Seitenwand eines Druckballes angeordnet ist. Das Einführen des Transfusionsbeutels in die sackartige Öffnung ist umständlich und zeitraubend. Eine Bluttransfusion unter Druck wird häufig als arterielle Transfusion insbesondere bei lebensbedrohlichem Blutverlust eines Patienten durchgeführt. Es hat sich dabei gezeigt, daß Situationen auftreten können, in der ein sofortiger Abbruch der Transfusion angezeigt ist, beispielsweise eine sich abzeichnende Unverträglichkeitsreaktion des Patienten oder wenn in der Hektik der ersten Notversorgung größere Mengen Luft in das zu dem Patienten führende Schlauchleitungssystem gelangt sind. Wenn in einem solchen Fall die Transfusion bei dem Behälter der herkömmlichen Art unterbrochen werden soll, ist dies nur dadurch möglich, daß der Druckball entlüftet wird. Diese Entlüftung nimmt jedoch sehr wertvolle Zeit in Anspruch.

Das Dokument EP-A-0102012 beschreibt eine Beutelpresseinheit, die aus einer Gesamthülse (oder Lasche) besteht, die mit einer Kante an einer flexiblen, eine starre Grundplatte umgebenden Hülse befestigt ist

Aus den Unterlagen des deutschen Gebrauchsmusters 19 20 114 ist es bekannt, bei einer Anordnung zur Transfusion von Blut unter Druck den Druckball mit einem Entlüftungsstutzen zu versehen. Hierdurch kann zwar eine schnellere Entlüftung des Druckballes oder der Druckmanschette im Hinblick auf einen bevorstehenden Wechsel des Transfusionsbeutels erzielt werden. Gleichwohl nimmt jedoch auch bei dieser bekannten Lösung die Entlüftung und Entspannung der Druckmanschette wertvolle Sekunden in Anspruch und ist insbesondere nicht geeignet, in einem der oben geschilderten Notfälle die Transfusion schlagartig zu unterbrechen.

Der Erfindung liegt die Aufgabe zugrunde, einen Behälter der eingangs genannten Art zu schaffen, bei dem der Infusions/Transfusions-Beutel rasch ausgewechselt werden kann und der insbesondere eine möglichst rasche Unterbrechung derInfusion/Transfusion ermöglicht.

Diese Aufgabe ist bei der Vorrichtung gemäß Anspruch 1 gelöst.

Durch die Lasche werden beim Durchführen der Infusion/Transfusion unter Druck das Druckkissen und der Infusions/Transfusions-Beutel zusammengepreßt, so daß die Infusions/Transfusions-Flüssigkeit unter Druck in den Körper des Patienten eingeführt wird. Soll die Infusion/Transfusion plötzlich unterbrochen werden, so ist lediglich der Verschluß zu öffnen, worauf die Preßverbindung zwischen Druckkissen und Infusions/Transfusions-Beutel aufgehoben und der Infusions/Transfusions-Beutel sofort entspannt wird. Der Infusions/Transfusions-Beutel kann somit auch sofort ausgewechselt werden, ohne daß die vollständige oder zumindest weitgehende Entlüftung des Druckkissens abgewartet werden muß. Das Einführen eines neuen Beutels ist einfach. Hierzu wird dieser lediglich auf das Druckkissen gelebt. Danach wird die Lasche zu einem etwa rohrförmigen Behälter zusammengelegt und der Verschluß geschlossen. Ein zeitraubendes Einführen des Infusions/Transfusions-Beutels in eine sackartige Aufnehmung entfällt.

Eine besonders vorteilhafte Ausführungsform der Erfindung ist dadurch gekennzeichnet, daß eine sackförmige, das Druckkissen aufnehmende Tasche vorgesehen ist, und daß die Lasche mit ihrem einen Ende an einer Längsseite der Tasche befestigt ist. Dadurch wird das Druckkissen in einer präzis definierten Position festgehalten, so daß zur Einleitung der Infusion/Transfusion unter Druck lediglich der Infusions/Transfusions-Beutel auf das Druckkissen gelegt werden muß. Danach wird noch die Lasche um den Beutel und das Druckkissen geschlagen und endseitig befestigt.

Nach einer besonders vorteilhaften Ausführungsform ist der Verschluß als (handelsüblicher) Klettverschluß ausgeführt. Klettverschlüsse können besonders schnell geschlossen und geöffnet werden. Durch eine entsprechende Bemessung der Fläche des Klettverschlusses kann auch eine hinreichende, dem Druck des Druckkissens standhaltende Schließkraft erzielt werden.

Vorteilhafterweise wird nach einer weiteren Ausführungsform der Erfindung ein Klettband in der Nähe des freien Endes der Lasche und das entsprechende als Gegenstück wirkende Klettband auf der Tasche in der Nähe des Bereichs angeordnet, an dem die Lasche befestigt ist. Somit kann die Lasche etwa einmal um den gesamten Behälter geschlagen werden. Ein besonders sicherer Halt hat sich ergeben, wenn mehrere Klettbandstreifen parallel zur Richtung der Linie vorgesehen werden, längs der die Lasche an der Tasche befestigt ist. Ein rasches Öffnen des Verschlusses wird gemäß einer weiteren vorteilhaften Ausführungsform dadurch begünstigt, daß die Lasche ein klettbandfreies Griffteil, d.h. einen Abschnitt aufweist, der frei von dem Klettbandverschluß ist, so daß die Lasche an diesem freien Griffteil ergriffen und der Verschluß im Bedarfsfall rasch aufgerissen werden kann. Die Lasche besteht nach einer weiteren vorteilhaften Ausführungsform aus einem transparenten Material oder aus einem Netz, so daß der durch die Lasche bedeckte Infusions/Transfusions-Beutel sichtbar bleibt. Ein besonders sicherer Sitz des Infusions/Transfusions-Beutels wird erzielt, wenn die Lasche an ihren Rändern einen Saum aufweist. Der Saum ist weniger dehnbar als das Flächengewebe der Lasche. Durch den Saum wird der Infusionsbeutel an zwei Seiten eingeschnürt, so daß sich ein sicherer Sitz ergibt.

Die Tasche zur Aufnahme des Druckkissens ist mit einem Querschlitz zum Einführen des Druckkissens versehen. Dieser Querschlitz ist vorzugsweise mit einer flexiblen Klappe verschließbar. Dabei hat es sich als besonders vorteilhaft erwiesen, zu beiden Seiten des Querschlitzes jeweils Klettbandstreifen und einen entsprechenden Klettbandstreifen als Gegenhalteteil an der flexiblen Klappe anzuordnen. Vorteilhafterweise sind des weiteren zwei Löcher zum Durchführen von Luftschläuchen vorgesehen. Durch den einen der beiden Schläuche wird das Druckkissen üblicherweise durch ein Handgebläse aufgeblasen, während der zweite der beiden Schläuche an ein Manometer zur Überwachung des Druckes angeschlossen ist. Durch die Anordnung des Druckkissens in der oben beschriebenen Weise wird ein besonders sicherer Sitz desselben in dem Behälter erzielt. Die Einführung des Druckkissens erfolgt nicht unter Zeitdruck, vielmehr liegt vor Eintreten eines Notfalles der Behälter einschließlich des eingesetzten und angeschlossenen Druckkissens bereit.

In weiterer Ausgestaltung der Erfindung ist an der Tasche eine Schlinge zum Aufhängen des Behälters befestigt. Vorzugsweise kann durch diese Schlinge eine an der Lasche befestigte Aufhängeleine gezogen werden, die mit einer Öse an einem Ständer eingehängt werden kann.

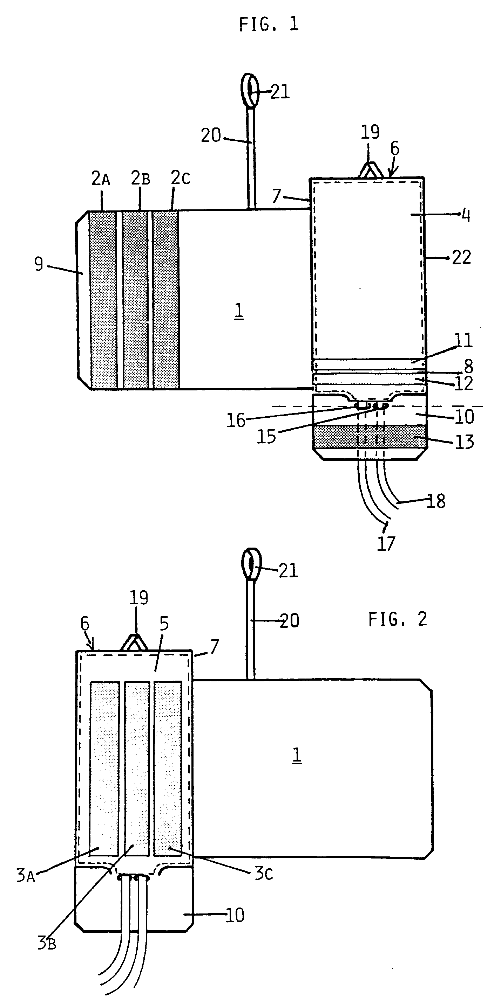

Die Erfindung wird im folgenden anhand des in den Figuren 1 und 2 dargestellten Ausführungsbeispieles näher erläutert. Es zeigt:

**Fig. 1:**     eine Ausführungsform eines Behälters mit aufgeklappter Lasche;

**Fig. 2:**     den Behälter gemäß Fig. 1, jedoch von der Rückseite gesehen.

Der Behälter gemäß der Erfindung besteht aus einem textilen, leicht reinigbaren Kunststoffmaterial. Zwei etwa rechteckige Teile 4 und 5 sind etwa deckungsgleich aufeinandergelegt und durch eine ringsum verlaufende Naht 22 zu einer Tasche 6 vernäht. An einer Schmalseite der rechteckigen Tasche 6, die im folgenden der Einfachheit halber als "obere Seite" bezeichnet wird, ist eine Aufhängeschlinge 19 angenäht. An der entgegengesetzten Schmalseite, die im folgenden der Einfachheit halber als "untere Seite" bezeichnet wird, ist die Naht 22 unterbrochen. Das Textilteil 4, das im folgenden der Einfachheit halber als "vorderes Textilteil" bezeichnet wird; weist in der Nähe der unteren schmalen Seite einen Querschlitz 8 auf. Zu beiden Seiten des Querschlitzes sind zu diesem parallel verlaufende Klettstreifen 11, 12 angenäht. Das Textilteil 5, das im folgenden der Einfachheit halber, als "hinteres Textilteil" bezeichnet wird, ragt an der unteren Seite etwas über die untere Seite des vorderen Textilteiles 4 hinaus, so daß eine Klappe 10 gebildet wird, an der ein zu den Klettbändern 11 und 12 komplementäres Klettband 13 angenäht ist. In der Klappe 10 sind zwei Löcher 15, 16 vorgesehen. An der Längsseite 7 der Tasche 6 ist eine etwa rechteckige Lasche 1 angenäht, deren Breite etwa der Länge der rechteckigen Tasche 6 entspricht. Die Tasche 1 besteht aus einem transparenten Gewebe geringer Dehnungsfähigkeit. Parallel zur Längskante 7 der Tasche 6 sind auf der Lasche 1 drei Klettbandstreifen 2a, 2b, 2c angenäht. Die Klettbandstreifen 2a, 2b, 2c halten vom längsseitigen Endrand der Lasche 1 einen Abstand ein, so daß dort ein freier Rand 9 bestehen bleibt. Zu den Klettbandstreifen 2a, 2b, 2c komplementäre Klettbandstreifen 3a, 3b, 3c sind in entsprechender Form auf dem hinteren Textilteil 5 der Tasche 6 angenäht, wie in Fig. 2 zu erkennen ist. An der oberen Längsseite der Lasche 1 ist eine Aufhängeleine 20 mit einer endseitigen Öse 21 befestigt.

Der Behälter wird wie folgt verwendet: in die aus den zusammengenähten Textilstücken 4 und 5 bestehende Tasche 6 wird durch den Schlitz 8 ein Druckkissen, an das zwei Sehläuche 17, 18 angeschlossen sind, eingeführt. Die Schläuche werden aus dem Inneren der Tasche durch die Löcher 15 bzw. 16 nach außen geführt. Einer der beiden Schläuche wird an ein Handgebläse, der andere an ein Manometer angeschlossen. Darauf wird die Klappe 10 umgefaltet, derart, daß das Klettband 13 auf die beiden Klettbandstreifen 11 und 12 zu beiden Seiten des Schlitzes 8 kommt. Das Klettband wird dann angepreßt, so daß die Tasche 6 sicher verschlossen ist. In diesem Zustand liegt die Anordnung für den Notfall, beispielsweise eine arterielle Bluttransfusion, bereit.

Im Bedarfsfall wird beispielsweise ein Blutbeutel auf das Textilstück 4 der Tasche 6 derart gelegt, daß der zum Patienten führende Anschlußschlauch an der unteren Seite, d.h. an der der Schlinge 19 abgewandten Seite liegt. Sodann wird die Lasche 1 über den Blutbeutel geschlagen, so daß die Klettverschlußstreifen 2a, 2b, 2c zu den an der Rückseite der Tasche 6 angeordneten komplementären Klettverschlußstreifen 3c, 3b, 3a gelangen und durch Andruck mit diesen befestigt werden. Die Länge der Lasche 1 ist so bemessen, daß bei Einschluß eines normalen Blutbeutels, das freie Ende der Lasche 1 an der Rückseite der Tasche 6 etwa im Bereich der Längsseite der Tasche 6 liegt, so daß die Lasche sowohl den Blutbeutel als auch das Druckkissen unter Bildung eines etwa rohrförmigen, etwas ausgebauchten Behälters umschließt. Es wird dann die Leine 20 mit ihrer Öse 21 durch die Schlinge 19 gezogen und die aus dem Behälter, dem Blutbeutel und dem Druckkissen bestehende Anordnung wird mittels der Öse 21 an einem Ständer aufgehängt. Danach kann die Bluttransfusion unter Druck in der üblichen, bekannten Weise beginnen.

Da der Blutbeutel nicht in eine sackförmige Öffnung eingeführt werden muß, und da des weiteren der Anschlußschlauch des Blutbeutels nicht durch eine Öse geführt werden muß, kann das Einsetzen des Blutbeutels in Sekundenschnelle erfolgen. Es hat sich gezeigt, daß der Blutbeutel durch die Lasche 1 lediglich durch Andruck ausreichend sicher gehalten wird. Insbesondere Säume (nicht dargestellt) an den Längskanten der Lasche 1 schnüren den Blutbeutel ein, so daß ein Ausweichen nach oben oder unten unmöglich ist. Das Material der Lasche 1 ist nur von geringer Nachgiebigkeit, so daß der Blutbeutel nicht ausweichen kann, und dem Druck des Druckkissens ausgesetzt wird. Eine geringe Dehnungsfähigkeit des Materials der Lasche 1, die zu einer Ausbauchung ohne Beeinträchtigung des Druckaufbaues führt, ist jedoch unschädlich. Vielmehr wird durch diese geradezu ein sicheres Umschließen des Blutbeutels gewährleistet.

Die Haftfläche der Klettbandstreifen 2a, 2b, 2c bzw. ihrer Gegenhalteteile 3a, 3b, 3c ist ausreichend bemessen, so daß selbst dann ein hinreichend fester Sitz gewährleistet ist, wenn die Klettstreifen 2a, 2b, 2c nicht vollkommen deckungsgleich auf ihren komplementären Klettstreifen 3a, 3b, 3c zu liegen kommen. Dadurch, daß die Lasche 1 aus transparentem Material gefertigt ist, kann die Blutkonserve durch die Lasche 1 hindurch beobachtet werden.

**Patentansprüche**

1. Vorrichtung zur Durchführung einer Infusion/Transfusion unter Druck, mit einer Lasche (1), die mittels eines Verschlusses (2a, 2b, 2c, 3a, 3b, 3c) zu einem rohrförmigen, einen Infusions-/Transfusionsbeutel aufnehmenden Behältnis zusammenschließbar ist, und mit einem aufblasbaren Druckkissen zur Erhöhung des Druckes innerhalb des Behältnisses, wobei die Lasche (1) mit ihrem einen Ende an einer Längsseite einer sackförmigen Tasche (6) befestigt ist, und die Lasche (1) und die Tasche (6) aus textilem Material bestehen. **dadurch gekennzeichnet**, daß die Tasche (6) einen Querschlitz aufweist, und daß das aufblasbare Druckkissen in die Tasche (6) durch den Querschlitz (8) einführbar ist.

2. Vorrichtung nach Anspruch 1, dadurch gekennzeichnet, daß der Verschluß (2a, 2b, 2c, 3a, 3b, 3c) ein Klettverschluß ist.

3. Vorrichtung nach Anspruch 2, dadurch gekennzeichnet, daß mindestens ein Klettband (2a, 2b, 2c) in der Nähe des freien Endes der Lasche (1) und das entsprechende als Gegenstück wirkende Klettband (3a, 3b, 3c) auf der Tasche (6) in der Nähe des Bereichs angeordnet ist, an dem die Lasche (1) befestigt ist.

4. Vorrichtung nach Anspruch 3, dadurch gekennzeichnet, daß das oder die Klettbänder (2a, 2b, 2c, 3a, 3b, 3c) parallel zu der Linie verlaufen, längs der die Lasche (1) an der Tasche (6) befestigt ist.

5. Vorrichtung nach einem der Ansprüche 2 bis 4, dadurch gekennzeichnet, daß die Lasche (1) ein klettbandfreies Griffteil (9) aufweist.

6. Vorrichtung nach einem der Ansprüche 1 bis 5, dadurch gekennzeichnet, daß die Losche (1) aus transparentem Material besteht.

7. Vorrichtung nach einem der Ansprüche 1 bis 6, dadurch gekennzeichnet, daß die Lasche (1) aus einem Netz besteht.

8. Vorrichtung nach einem der Ansprüche 1 bis 7, dadurch gekennzeichnet, daß die Lasche (1) an ihren Außenrändern einen Saum aufweist.

9. Vorrichtung nach einem der Ansprüche 1 bis 8, dadurch gekennzeichnet, daß der Querschlitz (8) mittels einer flexiblen Klappe (10) verschließbar ist.

10. Vorrichtung nach Anspruch 9 und 10, dadurch gekennzeichnet, daß zu beiden Seiten des Querschlitzes (8) jeweils Klettbondstreifen (11, 12) und ein entsprechender Klettbondstreifen (13) als Gegenholteteil an der flexiblen Klappe (10) angeordnet sind.

11. Vorrichtung nach einem der Ansprüche 1 bis 10, dadurch gekennzeichnet, daß die Tasche (6) zwei Löcher (15, 16) zum Durchführen von Luftschläuchen aufweist.

12. Vorrichtung nach einem der Ansprüche 1 bis 11, dadurch gekennzeichnet, daß an der Tasche (6) eine Schlinge (19) befestigt ist.

13. Vorrichtung nach einem der Ansprüche 1 bis 12, dadurch gekennzeichnet, daß an der Losche (1) eine Aufhängeleine (20) befestigt ist, an deren freiem Ende eine Öse oder Schlaufe (21) angeordnet ist.

## Claims

1. Apparatus for performing an infusion/transfusion under pressure including a flap (1) which may be connected by means of a fastener (2a,2b,2c,3a,3b,3c) to form a tubular container receiving an infusion/transfusion bag and including an inflatable pressure pad for increasing the pressure within the container, whereby one end of the flap (1) is secured to a longitudinal side of a sack-shaped pouch (6) and the flap (1) and the pouch (6) comprise textile material, characterised in that the pouch (6) has a transverse slit and that the inflatable pressure pad may be inserted into the pouch (6) through the transverse slit (8).

2. Apparatus as claimed in claim 1, characterised in that the fastener (2a,2b,2c,3a,3b,3c) is a hook and loop fastener.

3. Apparatus as claimed in claim 2, characterised in that at least one hook and loop strip (2a,2b,2c) is disposed in the vicinity of the free end of the flap (1) and the corresponding hook and loop strip (3a,3b,3c), which acts as the complementary member, is disposed on the pouch (6) in the vicinity of the region to which the flap (1) is secured.

4. Apparatus as claimed in claim 3, characterised in that the hook and loop strip(s) (2a,2b,2c,3a,3b,3c) extend parallel to the line along which the flap (1) is secured to the pouch (6).

5. Apparatus as claimed in one of claims 2 to 4, characterised in that the flap (1) has a gripping portion (9) which has no hook and loop strip.

6. Apparatus as claimed in one of claims 1 to 5, characterised in that the flap (1) comprises transparent material.

7. Apparatus as claimed in one of claims 1 to 6, characterised in that the flap (1) comprises a gauze.

8. Apparatus as claimed in one of claims 1 to 7, characterised in that the flap (1) has a selvage.

9. Apparatus as claimed in one of claims 1 to 8, characterised in that the transverse slit (8) may be closed by means of a flexible flap (10).

10. Apparatus as claimed in claims 9 and 10, characterised in that disposed on both sides of the transverse slit (8) are respective hook and loop strips (11,12) and a corresponding hook and loop strip (13) is disposed as a complementary retaining member on the flexible flap (10).

11. Apparatus as claimed in one of claims 1 to 10, characterised in that the pouch (6) has two holes (15,16) through which air hoses may be passed.

12. Apparatus as claimed in one of claims 1 to 11, characterised in that a loop (19) is secured to the pouch (6).

13. Apparatus as claimed in one of claims 1 to 12, characterised in that a suspension cord (20), on whose free end there is an eye or loop (21), is secured to the flap (1).

## Revendications

1. Dispositif pour la réalisation d'une infusion/transfusion sous pression, avec une sangle (1), qui peut être réunie à un conteneur tubulaire accueillant un récipient d'infusion/transfusion, au moyen d'une fermeture (2a, 2b, 2c, 3a, 3b, 3c), et avec un coussin de pression gonflable pour l'élévation de la pression à l'intérieur du conteneur, à l'occasion de quoi la sangle (1) est fixée avec une de ses extrémités au grand côté d'une poche (6) en forme de sachet, la sangle (1) et la poche (6) étant constituées d'un matériau textile,
caractérisé en ce que la poche (6) présente une entaille transversale (8) et en ce que le coussin de pression gonflable peut être introduit dans la poche (6) à travers l'entaille transversale (8).

2. Dispositif selon la revendication 1, caractérisé en ce que la fermeture (2a, 2b, 2c, 3a, 3b, 3c) est une bande agrippante.

3. Dispositif selon la revendication 2, caractérisé en ce qu'au moins une bande agrippante (2a, 2b, 2c) est disposée à proximité de l'extrémité libre de la sangle (1) et en ce que la bande agrippante opérante homologue correspondante (3a, 3b, 3c) est disposée sur la poche (6) à proximité de la zone à laquelle est fixée la sangle (1).

4. Dispositif selon la revendication 3, caractérisé en ce que la ou les bande(s) agrippante(s) (2a, 2b, 2c, 3a, 3b, 3c) est (sont) disposée(s) parallèle(s) à la ligne le long de laquelle la sangle (1) est fixée à la poche (6).

5. Dispositif selon l'une des revendications 2 à 4, caractérisé en ce que la sangle (1) présente une partie pour la préhension (9), sans bande agrippante.

6. Dispositif selon l'une des revendications 1 à 5, caractérisé en ce que la sangle (1) est constituée d'un matériau transparent.

7. Dispositif selon l'une des revendications 1 à 6, caractérisé en ce que la sangle (1) est constituée d'un filet.

8. Dispositif selon l'une des revendications 1 à 7, caractérisé en ce que la sangle (1) présente un ourlet à sa bordure extérieure.

9. Dispositif selon l'une des revendications 1 à 8, caractérisé en ce que l'entaille transversale (8) peut être fermée au moyen d'un abattant flexible (10).

10. Dispositif selon les revendications 9 et 10, caractérisé en ce que des bandes agrippantes (11, 12) sont respectivement disposées de part et d'autre de l'entaille transversale (8) et en ce qu'une bande agrippante (13) correspondante est disposée sur l'abattant flexible (10) en tant que partie homologue pour le maintien.

11. Dispositif selon l'une des revendications 1 à 10, caractérisé en ce que la poche (6) présente deux ouvertures (15, 16) pour l'introduction de tuyaux flexibles à l'air.

12. Dispositif selon l'une des revendications 1 à 11, caractérisé en ce qu'une boucle (19) est fixée à la poche (6).

13. Dispositif selon l'une des revendications 1 à 12, caractérisé en ce qu'un cordon de suspension (20) est fixé à la sangle (1) à l'extrémité libre duquel est disposé un anneau ou un neud coulant (21).

FIG. 1

FIG. 2